# EUROPÄISCHE PATENTANMELDUNG

(11) **EP 1 512 459 A1**
(43) Veröffentlichungstag der Anmeldung: **09.03.2005**
(21) Anmeldenummer: 04020593.2
(22) Anmeldetag: 31.08.2004
(51) Int. Cl.: B01J 25/02, C07C 209/36

(54) **Verfahren und Herstellung von Raney-Nickel-Katalysatoren und deren Verwendung zur Hydrierung organischer Verbindungen**

(30) Priorität: 08.09.2003 DE 10341269
(71) Anmelder: Bayer MaterialScience AG, 51368 Leverkusen (DE)
(72) Erfinder: Zechlin, Joachim, Dr., 41472 Neuss (DE); Wegener, Gerhard, Dr., 40822 Mettmann (DE); Warlimont, Hans, Prof., Dr., 01326 Dresden (DE)

(57) **Zusammenfassung**

Die Erfindung betrifft ein Verfahren zur Herstellung von Raney-Nickel-Katalysatoren, bei dem man die Schmelze einer Legierung, enthaltend 40 bis 95 Gew.-% Aluminium, 5 bis 50 Gew.-% Nickel, sowie 0 bis 20 Gew.-% Eisen, 0 bis 15 Gew.-% Cer, Cer-Mischmetall, Vanadium, Niob, Tantal, Chrom, Molybdän und/oder Mangan sowie gegebenenfalls weitere glasbildende Elemente mit einer oder mehreren rotierenden Kühlwalzen oder Kühlscheiben in Kontakt bringt, darauf abkühlen und erstarren lässt, wobei die Kühlwalzen eine durch Querrillen und die Kühlscheiben eine durch von der Rotationsachse nach außen verlaufende Rillen strukturierte Oberfläche aufweisen, und man die rasch erstarrte Legierung anschließend einer Behandlung mit organischen oder anorganischen Basen unterzieht.

## Beschreibung

Die Erfindung betrifft ein Verfahren zur Herstellung von Raney-Nickel-Katalysatoren mit hoher Standzeit und hoher Produkt-Selektivität, und die Verwendung derart hergestellter Raney-Nickel Katalysatoren bei der Hydrierung organischer Verbindungen, insbesondere bei der Hydrierung aromatischer Nitroverbindungen.

Die Herstellung und Anwendung von Raney-Nickel als Hydrierkatalysator von aromatischen Nitroverbindungen wie z.B. Nitrobenzol, Nitrotoluolen, Dinitrotoluolen, chlorierten Nitroaromaten und anderen ist bekannt und öfters beschrieben worden (z.B. *R Schröter, Angew. Chem. 1941, **54***, *229 oder EP-A-0 223 035*). Üblicherweise geht man bei der Herstellung von Raney-Nickel-Katalysatoren von einer Vorlegierung aus Aluminium und Nickel und gegebenenfalls einem oder mehreren weiteren Nebengruppenmetallen als Katalysatorvorläufer aus. Die Legierung wird beispielsweise durch Schmelzen oder Reaktivmahlen der Ausgangsmetalle erhalten. Raney-Nickel-Katalysatoren lassen sich durch Legieren der Ausgangslegierung mit anderen Metallen zur Verbesserung der Aktivität, Selektivität und Stabilität insbesondere bei erhöhten Temperaturen modifizieren. Diese Dotierung des Katalysators durch Zusatz verschiedenster Metalle zur Al-Ni Schmelze der Katalysatorvorläufer ist bekannt (z.B. DE-A 40 01 484, DE-A 35 37 247). Die Katalysatorvorläufer werden durch Verdüsung der Al-Ni-Metallschmelze hergestellt oder gegossen und anschließend mechanisch zerkleinert. Der Katalysator wird dann durch teilweise oder vollständige Auslaugung des Aluminiums aus der Legierung mit einer Base freigesetzt (DE-A 27 13 374). Die katalytische Wirkung der aus den Legierungen hervorgehenden Katalysatoren hängt u.a. von der qualitativen und quantitativen Zusammensetzung der Legierung, der Struktur und dem Gefüge der Legierung und damit von der resultierenden Struktur und dem resultierenden Gefüge des Katalysators ab.

Die Hydrierung von aromatischen Nitroverbindungen ist eine großtechnisch häufig durchgeführte Reaktion. Hierfür werden vielfach Raney-Nickel-Katalysatoren eingesetzt. Katalysatorstandzeiten, Produkt-Selektivität, Strukturen und Gefüge der Ausgangslegierungen sowie die Erstarrungsgeschwindigkeit sind kaum korreliert. Insbesondere bei den ternären Systemen Al-Ni-Zusatzmetall kann in der Ausgangslegierung eine Vielzahl von Phasen vorliegen, die im resultierenden Katalysator keine oder nur geringe Aktivitäten, hohe Katalysatorverbräuche und geringe Produkt-Selektivitäten aufweisen. In DE-A 19 753 501 wird der Einsatz von amorphen, teilamorphen oder feinkristallinen, durch Rascherstarrung hergestellten Legierungen für die Herstellung von RaNi-Katalysatoren zur Erhöhung der Katalysator-Standzeit und somit zur Senkung des Katalysator-Verbrauches beschrieben. Als Herstellverfahren für die Vorlegierung sind dort das Ausgießen einer Metall-Schmelze auf eine rotierende Kühlwalze oder in den Spalt zwischen zwei rotierenden Kühlwalzen sowie die Schmelz-Extraktion beschrieben.

Hohe Abkühlgeschwindigkeiten von 10⁴ bis 10⁷ K/s lassen sich gemäß *A. Molnar, G. V. Smith, M. Bartok, Advances in Catalysis, **36**, 329-383 (1989)* durch Schmelz-Spinnen oder durch die Herstellung von Metall-Bändern erzielen. Dies kann z.B. durch Ausdrücken einer Legierungsschmelze auf eine oder in den Spalt zwischen zwei rotierenden Kühlwalzen geschehen oder durch den Ausguss auf eine gekühlte rotierende Scheibe sowie durch Verfahren, die dem Fachmann als Schmelzextraktionsverfahren (melt extraction rapid solidification technology, MERST) oder als Melt-Overflow-Verfahren (melt overflow rapid solidification technology, MORST) bekannt sind.

Bei der Melt-Overflow-Technologie fließt eine Metall-Schmelze in dünner Schicht im Allgemeinen über eine horizontale Überlauf-Kante auf eine rotierende Kühlwalze, wobei sich rascherstarrte Metall-Fasern bzw. Metall-Flocken bilden. Der Überlauf der Schmelze auf die Walze wird z.B. ermöglicht durch Kippen des Schmelztiegels, kann aber auch durch Verdrängung der Schmelze mit einem in den Schmelztiegel tauchenden Stempel erfolgen. Durch Schrumpfung der Legierung beim Abkühlen auf der kalten Metall-Oberfläche und durch die Fliehkraft der rotierenden Walze werden die Flocken oder Fasern von deren Oberfläche weggeschleudert. Das Melt-Overflow-Verfahren kann unter Luft erfolgen, unter einem Inertgas oder aber in einer Vakuum-Kammer.

Die Technologie zur Herstellung der Vorlegierung mit dem Melt-Overflow-Verfahren ist unter anderem beschrieben in *US-A-5 170 837* sowie in *US-A-4 907 641.*

Bei der Schmelzextraktion berührt eine oberhalb des Schmelztiegels angebrachte rotierende Kühlwalze die Oberfläche der Metallschmelze und zieht durch die Rotation rascherstarrte Metall-Fasern aus der Schmelze. Durch Schrumpfung der Legierung beim Abkühlen auf der kalten Metall-Oberfläche und durch die Fliehkraft der rotierenden Walze werden die Flocken oder Fasern von deren Oberfläche weggeschleudert.

Die Schmelzextraktion kann unter Luft erfolgen, unter einem Inertgas oder aber in einer Vakuum-Kammer.

Die Technologie zur Herstellung der Vorlegierung mit dem Schmelzextraktions-Verfahren ist unter anderem beschrieben in *O. Andersen, G. Stephani, Metal Powder Report,* ***54***, *30-34 (1999).*

Eine weitere Methode zur Rascherstarrung ist der Guss einer Metallschmelze auf eine rotierende Kühlscheibe, wobei die rascherstarrte Legierung tangential von der Scheibe geschleudert wird.

Der Guss auf die Kühlscheibe kann unter Luft erfolgen, unter einem Inertgas oder aber in einer Vakuum-Kammer.

Bei der Melt-Overflow-Technologie, der Schmelzextraktions-Technologie sowie beim Ausguss auf rotierende Walzen oder eine rotierende Scheibe können gemäß *A. Molnar, G. V. Smith, M. Bartok, Advances in Catalysis,* ***36***, *329-383 (1989)* Abkühlraten realisiert werden, die sehr viel größer als 10⁴ K/ s sind. Im Gegensatz zu konventionellen Rascherstarrungsverfahren wie die Verdüsung einer Metallschmelze in Wasser, wie sie z.B. in *EP-A-0 437 788* beschrieben wird, ist bei solchen Prozessen die Bildung eines unerwünschten Oxid-Gehaltes weitgehend unterbunden.

Beim Guss einer Metall-Schmelze auf oder in den Spalt zwischen zwei rotierenden Kühlwalzen, beim Melt-Overflow-Verfahren, beim Schmelzextraktionsverfahren oder beim Guss einer Metall-Schmelze auf eine rotierende Kühlscheibe werden bei der Herstellung von duktilen Metall-Legierungen üblicherweise Endlos-Fasern oder Endlos-Bänder hergestellt. Bei der Herstellung solcher Endlos-Fasern und -Bändern werden Kühlwalzen oder Kühlscheiben mit Oberflächen eingesetzt, die keine Strukturierung quer zur Rotationsrichtung aufweisen. Hierbei wird die gesamte Oberfläche der Kühlwalze oder der Kühlscheibe für die Kühlung und Erstarrung der Schmelze verwendet und die Kapazität der Kühlung somit komplett genutzt. Dadurch kann eine sehr gleichmäßige hohe Erstarrungsgeschwindigkeit und die Ausbildung einer sehr homogenen Mikrokristallinität erzielt werden. Die Verwendung von Kühlwalzen und Kühlscheiben ohne Strukturierung quer zur Rotationsrichtung ist dabei insbesondere auch daher vorteilhaft, da sich solche Strukturen auf der Oberfläche der Kühlwalzen oder Kühlscheiben negativ auf die Mikrostruktur der erzeugten Legierungen auswirken können, da die Abkühlgeschwindigkeit am Anfang und am Ende derartiger Oberflächenstrukturen auf der Kühlwalze oder Kühlscheibe geringer ist als in der Mitte. Desweiteren werden duktile Metall-Legierungen vorteilhaft als Endlos-Fasern oder -Bänder hergestellt, da sich durch die gleichmäßige Erstarrungsgeschwindigkeit der Legierung auf der Kühlwalze oder Kühlscheibe die Bildung von nicht-rascherstarrten Agglomeraten weitestgehend unterdrücken lässt.

Raney-Nickel-Vorlegierungen mit hohem Aluminium-Gehalt sind jedoch nicht duktile sondern spröde Materialien. Daher entstehen bei der Rascherstarrung von RaNi-Vorlegierungen mit rotierenden Kühlwalzen oder Kühlscheiben, beispielsweise nach dem Melt-Overflow-Verfahren oder dem Schmelzextraktionsverfahren, nach dem Stand der Technik lange Bruchstücke von Fasern oder Bändern mit unregelmäßiger Länge. Diese Fasern und Bänder mit undefinierter Länge neigen im Produkt-Behälter, bei der Förderung und beim Transport zur Verhakung und Verfilzung, sind nicht rieselfähig und haben bei der Abfüllung und beim Transport eine ausgesprochen niedrige Schüttdichte.

Die Fasern oder Bänder der Vorlegierung müssen daher für die Förderung, für den Transport und die Weitererarbeitung durch einen nachgeschalteten Mahlungs-Prozess auf eine verarbeitbare und förderbare Größe gebracht werden. Dieser zusätzliche Prozessschritt erfordert einen zusätzlichen apparativen und energetischen Aufwand bei der Herstellung der Vorlegierung. Zudem wird durch den zusätzlichen Energie-Eintrag beim Mahlen das Metall-Gefüge der Vorlegierung und seine Mikrostruktur verändert. Diese mechanisch induzierte Rekristallisation wird z.B. in *J. Friedrich, U. Herr, K. Samwer, Journal of Applied Physics, 87*, *2464 (2000)* beschrieben. Wird auf die Mahlung der Vorlegierung jedoch verzichtet, so wird deutlich mehr Transportvolumen benötigt und die Förderung der verhakten und verfilzten Legierung z.B. mit einem Förderband, einer Förderschnecke oder mit einem Luftstrom wird erheblich erschwert oder unmöglich gemacht.

Es gibt daher Bedarf für ein einfaches und wirtschaftliches Verfahren zur Herstellung von rascherstarrten RaNi-Katalysatoren, die insbesondere als Katalysatoren für die Hydrierung von Nitroaromaten zu den entsprechenden Aminen mit hohen Standzeiten und Selektivitäten geeignet sind.

Die Aufgabe der vorliegenden Erfindung bestand daher darin, Raney-Nickel-Katalysatoren und ein einfaches und wirtschaftliches Verfahren zur ihrer Herstellung zur Verfügung zu stellen, bei dem die durch Rascherstarrung hergestellten Raney-Nickel-Vorlegierungen nicht verhaken oder verfilzen und ohne zusätzlichen Aufwand transportiert und weiterbehandelt werden können.

Es wurde jetzt gefunden, dass sich die Partikelgröße oder Fasergröße von Raney-Nickel-Vorlegierungen gegenüber den üblicherweise bei der Rascherstarrung erhaltenen Legierungen deutlich reduzieren lässt, wenn durch Querrillen strukturierte Kühlwalzen für das Ausgießen auf oder in den Spalt zwischen zwei Kühlwalzen, für die Schmelzextraktion oder für das Melt-Overflow-Verfahren eingesetzt werden oder wenn durch von der Rotationsachse nach außen verlaufende Rillen strukturierte Kühlscheiben für das Ausgießen auf ein rotierende Scheibe eingesetzt werden. Die erfindungsgemäß hergestellten Kurzfasern oder Kurz-Bänder weisen eine deutlich höhere Schüttdichte auf, sind rieselfähig, neigen nicht zur Verfilzung und lassen sich mit herkömmlichen Förder-Vorrichtungen wie z.B. Förderbändern, Förderschnecken problemlos transportieren. Überraschenderweise übersteigen die katalytischen Eigenschaften der erfindungsgemäß hergestellten Raney-Nickel-Katalysatoren sogar noch die katalytischen Eigenschaften der nach dem Stand der Technik durch Rascherstarrung auf nicht quer zur Rotationsrichtung oberflächenstrukturierten Kühlwalzen oder Kühlscheiben hergestellten Raney-Nickel-Katalysatoren. Dies zeigt sich beispielsweise bei der Hydrierung von Dinitrotoluol in einer gesteigerten Ausbeute zu Toluylendiamin und einer erhöhten Standzeit des Katalysators.

Die Erfindung betrifft daher ein Verfahren zur Herstellung von Raney-Nickel-Katalysatoren, bei dem man die Schmelze einer Legierung, enthaltend 40 bis 95 Gew.-% Aluminium, 5 bis 50 Gew.-% Nickel, sowie 0 bis 20 Gew.-% Eisen, 0 bis 15 Gew.-% Cer, Cer-Mischmetall, Vanadium, Niob, Tantal, Chrom, Molybdän und/oder Mangan sowie gegebenenfalls weitere glasbildende Elemente mit einer oder mehreren rotierenden Kühlwalzen oder Kühlscheiben in Kontakt bringt, darauf abkühlen und erstarren lässt, wobei die Kühlwalzen eine durch Querrillen und die Kühlscheiben eine durch von der Rotationsachse nach außen verlaufende Rillen strukturierte Oberfläche aufweisen, und man die rasch erstarrte Legierung anschließend einer Behandlung mit organischen oder anorganischen Basen unterzieht.

Dabei zeichnen sich die Querrillen auf der Oberfläche der Kühlwalzen dadurch aus, dass sie im Wesentlichen quer, das heißt in ihrer Hauptausdehnungsrichtung im Wesentlichen parallel zur Rotationsachse der Kühlwalze, verlaufen und einen Winkel von bis zu 45° mit der Rotationsachse einschließen.

Bevorzugt sind die Querrillen geradlinig, sie können aber auch beispielsweise leicht gekrümmt oder gebogen sein. Bevorzugt verlaufen die Querrillen dabei in Richtung der Rotationsachse der Kühlwalze über die gesamte Länge der Kühlwalze. Es sind aber auch Konstruktionen denkbar, bei denen die Querrillen nur über einen Teil der Walzenlänge verlaufen.

Die auf der Oberfläche der Kühlscheiben von der Rotationsachse nach außen verlaufenden Rillen können geradlinig, beispielsweise radial, oder auch gekrümmt, beispielsweise bogenförmig, ausgebildet sein. Bevorzugt verlaufen die Rillen von der Rotationsachse bis zum Rand der Kühlscheibe. Es sind aber auch Konstruktionen denkbar, bei denen die radialen oder bogenförmigen Rillen nur über einen Teil des Scheiben-Radius verlaufen. Für spezielle Legierungszusammensetzungen besonders geeignete Geometrien können dabei leicht durch Versuche ermittelt werden.

Unter Rillen werden dabei erfindungsgemäß alle Formen von Kanälen, länglichen Nuten oder sonstigen länglichen Einkerbungen in der Oberfläche verstanden. Die Rillen können dabei beliebige Formen einnehmen, beispielsweise dreieckigen, viereckigen, halbrunden oder halbovalen Querschnitt haben. Die Rillen haben dabei bevorzugt eine maximale Tiefe von 0,5 - 20 mm und eine maximale Breite von 0,5 - 20 mm. Besonders bevorzugt werden Rillen mit einer Tiefe von 1 - 5 mm und einer Breite von 1 - 10 mm eingesetzt.

Bei dem erfindungsgemäßen Verfahren werden Abkühlraten von > 10⁴ K/s realisiert.

Die Erfindung betrifft insbesondere ein Verfahren zur Herstellung von Raney-Nickel-Katalysatoren, bei dem man die Schmelze nach dem Melt-Overflow-Verfahren aus einem Schmelztiegel auf eine rotierende Kühlwalze abfließen und darauf abkühlen und erstarren lässt, wobei die Oberfläche der Kühlwalze durch Quer-Rillen strukturiert ist.

Die Erfindung betrifft weiterhin insbesondere ein Verfahren zur Herstellung von Raney-Nickel-Katalysatoren, bei dem man aus der Schmelze nach dem Schmelzextraktionsverfahren Teile der Schmelze durch eine in die Schmelze eintauchende rotierende Kühlwalze austrägt und darauf abkühlen und erstarren lässt, wobei die Oberfläche der Kühlwalze durch Quer-Rillen strukturiert ist.

Die Erfindung betrifft weiterhin insbesondere ein Verfahren zur Herstellung von Raney-Nickel-Katalysatoren, bei dem man die Schmelze auf eine rotierende Kühlwalze oder in den Spalt zwischen zwei gegensinnig rotierenden Kühlwalzen gießt und darauf abkühlen und erstarren lässt, wobei die Oberfläche der Kühlwalzen durch Quer-Rillen strukturiert ist.

Die Erfindung betrifft weiterhin insbesondere ein Verfahren zur Herstellung von Raney-Nickel-Katalysatoren, bei dem man die Schmelze auf eine rotierende Kühlscheibe gießt und darauf abkühlen und erstarren lässt, wobei die Oberfläche der Kühlscheibe durch von der Rotationsachse nach außen verlaufende Rillen strukturiert ist.

Durch das Abschrecken einer metallischen Schmelze mit hohen Abkühlraten sehr viel größer als 10⁴K/s lassen sich metastabile Phasen und Strukturen außerhalb des Gleichgewichtszustandes erhalten und einfrieren. Eine Verfeinerung des Gefüges, d.h. kleinere Kristallitgrößen, im Bereich von 1 - 10 µm und darunter, bevorzugt < 2 µm, werden so bei den erfindungsgemäßen Katalysatoren eingestellt. Erfolgt die Rascherstarrung der Legierungsschmelze mit sehr viel größer als 10⁴ K/s, so werden amorphe Legierungen oder Legierungen mit kristallinen und amorphen Bereichen, im weiteren als teilamorph bezeichnet, oder vollständig feinkristalline Zustände erhalten. Mit dem Begriff amorph im Zusammenhang mit metallischen Phasen, auch als metallische Gläser oder unterkühlte, feste Schmelzen klassifiziert, wird das Fehlen von Kristallinität beschrieben.

Die Ausbildung amorpher oder teilamorpher Strukturen kann zusätzlich durch Beifügung von weiteren Legierungsmetallen positiv beeinflusst werden. Als derartige Legierungsmetalle im erfindungsgemäßen Katalysator werden Seltenerd-Metalle, bevorzugt Cer oder Cer-Mischmetall sowie 0,5-1% Yttrium und/oder ausgewählte Nebengruppenelemente, bevorzugt Vanadium, Niob, Tantal, Chrom, Molybdän oder Mangan eingesetzt. Zudem können alternativ weitere glasbildende Hauptgruppenelemente, bevorzugt Bor, Silicium, Kohlenstoff und/oder Phosphor, enthalten sein.

Die erfindungsgemäßen Katalysatoren auf Basis amorpher/teilamorpher oder feinkristalliner durch Rascherstarrung nach der Melt-Overflow Technologie sowie nach der Schmelzextraktions-Technologie unter Einsatz erfindungsgemäß strukturierter Kühlwalzen oder durch Ausgießen der Legierung auf eine erfindungsgemäß strukturierte rotierende Scheibe oder Kühlwalze oder in den Spalt zwischen zwei solchen Kühlwalzen hergestellten Legierungen zeichnen sich im Vergleich zu herkömmlichen Raney-Nickel-Katalysatoren, deren Vorlegierungen nicht rascherstarrt wurden, durch eine deutlich erhöhte Produkt-Selektivität und Katalysator-Standzeit insbesondere bei hohen Reaktionstemperaturen > 120°C aus. Der Produkt-Ausstoß beim Einsatz der erfindungsgemäßen Katalysatoren zur Hydrierung von organischen Verbindungen im großtechnischen Maßstab wird dadurch erhöht und die erzeugten Abfallmengen werden deutlich reduziert. Dies kommt z.B. bei der technischen Herstellung von 2,4-/ 2,6-Toluylendiamin durch Hydrierung von Dinitrotoluolen oder anderen aromatischen Nitroverbindungen insbesondere in der fremdlösungsmittelfreien Hydrierung vorteilhaft zur Auswirkung.

Die nach den erfindungsgemäßen Verfahren hergestellten Katalysatoren zeichnen sich aber auch im Vergleich zu den nach dem Stand der Technik durch Rascherstarrung der Vorlegierung auf nicht quer zur Rotationsrichtung oberflächenstrukturierten Kühlwalzen oder Kühlscheiben hergestellten Raney-Nickel-Katalysatoren bei der Hydrierung von Nitroverbindungen durch erhöhte Ausbeute zu den entsprechenden Aminen und erhöhte Standzeit aus.

Für die erfindungsgemäße Herstellung der Katalysatoren aus rasch erstarrten Legierungen bzw. für die Herstellung amorpher, teilamorpher oder feinkristalliner Legierungen als Katalysatorvorläufer werden Legierungen enthaltend 40 bis 95 Gew.-% Aluminium, 5 bis 50 Gew.-% Nickel, 0 bis 20 Gew.-% Eisen sowie 0 bis 15 Gew.-% Cer, Cer-Mischmetall, Vanadium, Niob, Tantal, Chrom, Molybdän und/oder Mangan, eingesetzt. Bevorzugt sind Legierungen aus 50 bis 90 Gew.-% Aluminium, 15 bis 50 Gew.-% Nickel, 0 bis 10 Gew.-% Eisen sowie 0 bis 10 Gew.-% Cer, Cer-Mischmetall, Vanadium, Niob, Tantal, Chrom, Molybdän und/oder Mangan. Besonders bevorzugt sind Legierungen aus 60 bis 85 Gew.-% Aluminium, 15 bis 40 Gew.-% Nickel, 0 bis 6 Gew.-% Eisen sowie 0 bis 10 Gew.-% Cer, Cer-Mischmetall, Vanadium, Niob, Tantal, Chrom, Molybdän und/oder Mangan.

Diese Legierungs-Schmelzen können z.B. durch induktives Erschmelzen der Metalle in entsprechenden Gewichtsverhältnissen hergestellt werden.

Die Dicke der auf den Kühlwalzen oder Kühlscheiben erhaltenen Flakes oder Fasern liegt üblicherweise zwischen 10 und 150 µm, bevorzugt zwischen 20 und 120 µm, besonders bevorzugt zwischen 30 und 100 µm. Das Kühlrad besteht bevorzugt aus Cu, Ag oder Cu- und Ag-Basislegierungen, oder Edelstahl, kann aber auch aus beliebigen anderen metallischen Werkstoffen hergestellt werden. Kühlung der Kühlwalze ist möglich mit Raumluft, gekühlten Gasen, durch Wasser oder ein beliebiges anderes gasförmiges oder flüssiges Kühlmedium.

Die Freisetzung der Raney-Nickel-Katalysatoren wird durch Alkalibehandlung der gegebenenfalls zerkleinerten rasch erstarrten Legierung mit wässrigen Lösungen von organischen oder anorganischen Basen, beispielsweise mit Natrium- oder Kaliumhydroxid, Natrium- oder Kaliumcarbonat, bevorzugt mit Natrium- oder Kaliumhydroxid, vorzugsweise bei Temperaturen von 50 bis 150°C durchgeführt. Die Menge an Base richtet sich nach der Menge des in der Legierung vorliegenden Aluminiums. Die Base kann stöchiometrisch, im Überschuss oder Unterschuss zum Aluminium eingesetzt werden. Bevorzugt ist ein Mengenverhältnis von Aluminium zu Base von 1:1 bis 1:10, besonders bevorzugt ein Verhältnis von 1:1.1 bis 1:5. Der Katalysator kann durch teilweises oder vollständiges Dekantieren oder Abfiltrieren der wässrigen Lösung isoliert werden und durch wiederholtes Dekantieren oder Abfiltrieren von zugefügter Waschlösung gewaschen werden. Als Waschlösung wird Wasser (entionisiert, destilliert, Trinkwasser oder Brauchwasser) oder eine Lösung von Natrium- oder Kaliumhydroxid in Wasser verwendet.

Die durch Auslaugung aus den beschriebenen rasch erstarrten amorphen, teilamorphen oder feinkristallinen Legierungen herstellbaren Katalysatoren enthalten Restmengen von 0 bis 15 Gew.-% Aluminium, 50 bis 100 Gew.-% Nickel, 0 bis 50 Gew.-% Eisen sowie 0 bis 30 Gew.-% Cer, Cer-Mischmetall, Vanadium, Niob, Tantal, Chrom, Molybdän und/oder Mangan. Bevorzugt sind Katalysatoren aus 0 bis 10 Gew.-% Aluminium, 60 bis 100 Gew.-% Nickel, 0 bis 30 Gew.-% Eisen sowie 0 bis 30 Gew.-% Cer, Cer-Mischmetall, Vanadium, Niob, Tantal, Chrom, Molybdän und/oder Mangan. Besonders bevorzugt sind Legierungen aus 0 bis 10 Gew.-% Aluminium, 70 bis 100 Gew.-% Nickel, 0 bis 20 Gew.-% Eisen sowie 0 bis 25 Gew.-% Cer, Cer-Mischmetall, Vanadium, Niob, Tantal, Chrom, Molybdän und/oder Mangan.

Ein weiterer Gegenstand der Erfindung ist die Verwendung der beschriebenen Katalysatoren zur Hydrierung von organischen Verbindungen, bevorzugt zur Hydrierung aromatischer Nitroverbindungen und ein Verfahren zur Hydrierung aromatischer Nitroverbindungen unter Einsatz der erfindungsgemäß hergestellten Katalysatoren.

Als Ausgangsmaterialien eignen sich dabei beliebige aromatische Nitroverbindungen, z.B. Nitrobenzol, die Isomeren und deren Gemische von Nitrotoluol, chlorierten Nitroaromaten, Dinitronaphtalin und insbesondere die Isomeren und Isomerengemische des Dinitrotoluols. Die Nitroverbindungen werden vorzugsweise in Substanz, also ohne Verwendung eines Fremdlösungsmittels, bei Temperaturen von 100 bis 250°C, vorzugsweise von 120°C bis 200°C, und einem Druck von 5 bis 100 bar, vorzugsweise bei 10 bis 50 bar, in Gegenwart des im Reaktionsmedium suspendierten Katalysators hydriert. Das Reaktionsmedium setzt sich im Wesentlichen aus Produkt, entsprechend gebildetem Wasser und der Gasphase zusammen.

Dem Reaktionsmedium kann dabei gegebenenfalls auch ein Lösungsmittel, wie z.B. ein Alkohol, bevorzugt Methanol oder 2-Propanol, zugesetzt werden. Die Hydrierung anderer Nitroverbindungen erfolgt oft in einem Lösungsmittel, wie z.B. in einem Alkohol, bevorzugt in Methanol oder 2-Propanol, bei einem Druck von 5 bis 200 bar.

Die Hydrierung kann kontinuierlich oder diskontinuierlich in den üblichen Reaktoren erfolgen. Die Menge der in den Reaktor eingespeisten Nitroverbindung entspricht bei der kontinuierlichen Fahrweise der gleichzeitig aus dem Reaktor ausgeschleusten Menge an Reaktionsprodukt.

Überraschend ist die Erhöhung der Produktivität und die Senkung der Herstellkosten bei der Katalysator-Produktion mit dem erfindungsgemäßen Verfahren im Vergleich zu Katalysatoren aus Vorlegierungen, die durch Walzen-Rascherstarrung unter Einsatz von unstrukturierten Kühlwalzen entstehen. Die resultierende Erhöhung der Produktivität und Senkung der Herstellkosten für die Katalysatoren ist begründet in einer Erhöhung der Schüttdichte der rascherstarrten Vorlegierung, der verbesserten Förderbarkeit mit Förderbändern, Förderschnecken oder im Luftstrom, der Reduktion des notwendigen Frachtraumes beim Transport sowie in der Reduktion oder Elimierung des Mahl-Aufwandes solcher Legierungen.

Die in dem erfindungsgemäßen Verfahren eingesetzten Kühlwalzen bzw. Kühlscheiben werden anhand der folgenden Figuren näher erläutert.

Es zeigen
- Figur 1: eine glatte Kühlwalze, deren Oberfläche mit Querrillen strukturiert ist (Fig. 1 a) bzw. eine für Schmelz-Extraktion/ melt-overflow-Verfahren üblicherweise eingesetzte längs-strukturierte Kühlwalze, deren Oberfläche mit Querrillen strukturiert ist (Fig. 1b).
- Figur 2: eine Kühlscheibe mit radial verlaufenden Rillen
- Figur 3: eine Draufsicht auf eine Kühlscheibe mit radial (Fig. 3a) bzw. bogenförmig (Fig. 3b) verlaufenden Rillen

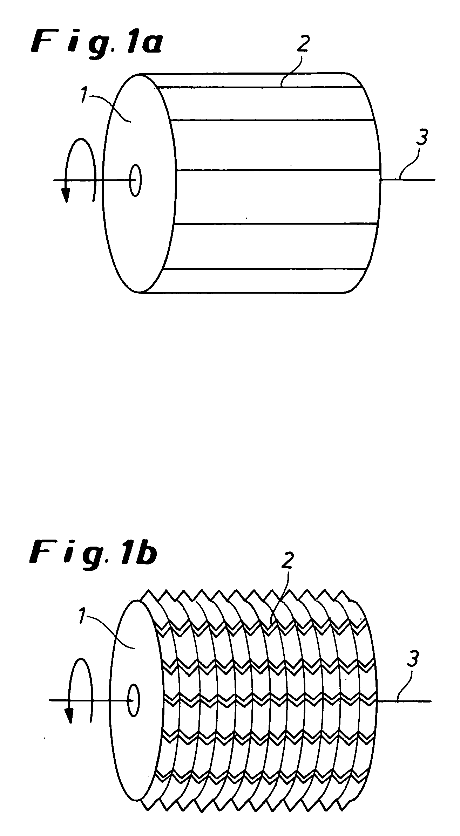

In den Figuren 1a und 1b ist eine rotierende Kühlwalze 1 gezeigt, die auf ihrer Oberfläche parallel zur Rotationsachse 3 verlaufende Querrillen 2 aufweist. Die Querrillen 2 erstrecken sich dabei in Richtung der Rotationsachse 3 über die gesamte Länge der Kühlwalze 1.

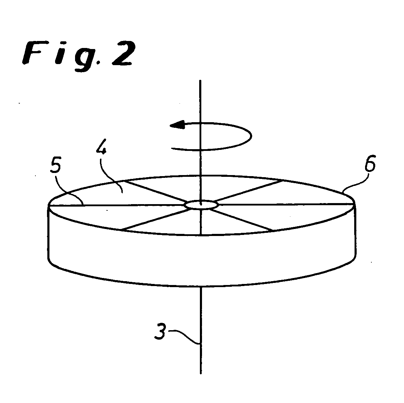

Figur 2 zeigt eine Kühlscheibe 4 mit von der Rotationsachse 3 nach außen bis zum Rand 6 verlaufenden radialen Rillen 5.

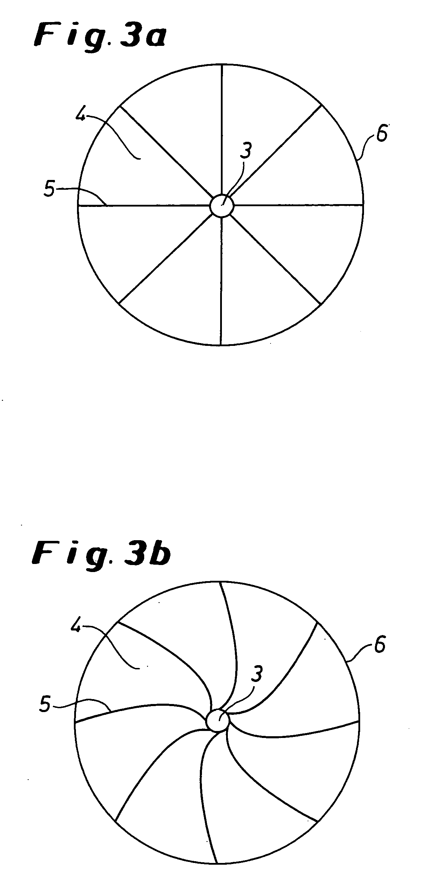

In Figur 3 ist eine Draufsicht auf eine erfindungsgemäß eingesetzte Kühlscheibe 4 mit radial von der Rotationsachse 3 bis zum Rand 6 verlaufenden Rillen 5 (Fig. 3a) bzw. bogenförmig verlaufenden Rillen 5 (Fig. 3b) gezeigt.

### Beispiele

### Herstellung von Vorlegierungen

### Beispiel 1 (erfindungsgemäß)

Herstellung der Vorlegierung Al80Ni17Fe3 durch Rascherstarrung nach dem Melt-Overflow-Verfahren mit einer oberflächenstrukturierten Kühlwalze.

Die für die Herstellung von 20 kg notwendigen Mengen an Aluminium, Nickel und Eisen wurden in einem Induktionsofen erschmolzen und anschließend in einen induktiv beheizten Tiegel eingefüllt. Die Schmelze wurde unter Luft über eine horizontale Ablaufkante auf eine aufwärts rotierende strukturierte Kühlwalze geführt (Querrillen-Abstand 30 mm). Die sich von der Kühl-Oberfläche lösenden und nach oben wegfliegenden Legierungs-Partikel mit einer Länge von ca. 30 mm und einer Dicke von ca. 0,1 mm wurden gesammelt.

### Beispiel 1b (erfindungsgemäß)

Herstellung der Vorlegierungen A171Ni26Fe3 durch Rascherstarrung nach dem Melt-Overflow-Verfahren mit einer oberflächenstrukturierten Kühlwalze.

Die für die Herstellung von 20 kg notwendigen Mengen an Aluminium, Nickel und Eisen wurden in einem Induktionsofen erschmolzen und anschließend in einen induktiv beheizten Tiegel eingefüllt. Die Schmelze wurde unter Luft über eine horizontale Ablaufkante auf eine aufwärts rotierende strukturierte Kühlwalze geführt (Querrillen-Abstand 30 mm). Die sich von der Kühl-Oberfläche lösenden und nach oben wegfliegenden Legierungs-Partikel mit einer Länge von ca. 30 mm und einer Dicke von ca. 0,1 mm wurden gesammelt.

### Beispiel 2 (erfindungsgemäß)

Herstellung der Vorlegierung Al80Ni17Fe3 durch Rascherstarrung nach dem Schmelzextraktionsverfahren mit einer oberflächenstrukturierten Kühlwalze.

Die für die Herstellung von 10 kg notwendigen Mengen an Aluminium, Nickel und Eisen wurden in einem Induktionsofen erschmolzen und anschließend in einen induktiv beheizten Tiegel eingefüllt. Durch Eintauchen einer rotierenden strukturierten Kühlwalze (Querrillen-Abstand 30 mm) wurden unter Luft Fasern einer Länge von ca. 30 mm und einer Dicke von ca. 0,1 mm aus der Schmelze gezogen. Die sich von der Kühl-Oberfläche lösenden in Rotationsrichtung wegfliegenden Legierungs-Partikel wurden gesammelt.

### Beispiel 3 a (nicht erfindungsgemäß)

Herstellung der Vorlegierung Al80Ni17Fe3 durch Rascherstarrung durch Guss auf eine Kühlwalze ohne Oberflächenstruktur.

Die Vorlegierung wurde aus 8000 g Aluminium, 1700 g Nickel und 300 g Eisen im Induktionsofen im Aluminiumoxid-Tiegel erschmolzen und in eine Kupferkokille abgegossen (Gesamtmenge Legierung: 10 kg). Die in Stangenform vorgeschmolzenen Ingots wurden in den Tiegel mit angeschmolzener Düse einer Rascherstarrungs-Anlage eingesetzt. Das induktive Aufschmelzen der Vorlegierung erfolgt in ca. 2 min mit einer Überhitzung von ca. 150°C. Die Schmelze wurde mittels Argon durch 0.1 mm auf eine rotierende, wassergekühlte Kupferwalze ohne Oberflächenstruktur ausgepresst. Das sich nach erfolgter Erstarrung von der Walzen-Oberfläche lösende Legierungs-Band wurde aufgefangen.

### Beispiel 3 b (nicht erfindungsgemäß)

Herstellung der Vorlegierung Al80Ni17Fe3 durch Rascherstarrung durch Guss auf eine Kühlwalze ohne Oberflächenstruktur.

Die Vorlegierung wurde aus 800 g Aluminium, 170 g Nickel und 30 g Eisen im Induktionsofen erschmolzen und in einer Rascherstarrungs-Anlage eingesetzt. Das induktive Aufschmelzen der Vorlegierung erfolgt mit einer Überhitzung von ca. 100-150°C. Die Schmelze wurde mittels Argon durch eine Schlitz-Düse auf eine rotierende, wassergekühlte Kupferwalze ohne Oberflächenstruktur ausgepresst. Das sich nach erfolgter Erstarrung von der Walzen-Oberfläche lösende Legierungs-Band wurde aufgefangen.

### Beispiel 4 (erfindungsgemäß)

Herstellung der Vorlegierung Al80Ni17Fe3 durch Rascherstarrung durch Guss auf eine oberflächenstrukturierte Kühlwalze.

Die für die Herstellung von 60 kg Al80Ni17Fe3-Schmelze notwendigen Mengen an Aluminium, Nickel und Eisen wurden in einem Induktionsofen erschmolzen und anschließend in einen beheizbaren Tiegel eingefüllt. Durch Düsen im Boden des Tiegels wurde die Metall-Schmelze auf eine rotierende wassergekühlte Kühlwalze gegossen (Querrillen-Abstand 30 mm), die sich im Innern einer Vakuum-Kammer befmdet. Dabei wurden durch Rascherstarrung Legierungs-Partikel einer Länge von ca. 30 mm und einer Dicke von ca. 0,1 mm gebildet. Die sich von der Kühl-Oberfläche lösenden in Rotationsrichtung wegfliegenden Legierungs-Partikel wurden gesammelt.

### Beispiel 5 (erfindungsgemäß)

Herstellung der Vorlegierung Al71Ni26Fe3 durch Rascherstarrung durch Guss auf eine oberflächenstrukturierte Kühlwalze.

Die für die Herstellung von 60 kg Al71Ni26Fe3-Schmelze notwendigen Mengen an Aluminium, Nickel und Eisen wurden in einem Induktionsofen erschmolzen und anschließend in einen beheizbaren Tiegel eingefüllt. Durch Düsen im Boden des Tiegels wurde die Metall-Schmelze auf eine rotierende wassergekühlte Kühlwalze gegossen (Querrillen-Abstand 30 mm), die sich im Innern einer Vakuum-Kammer befindet. Dabei wurden durch Rascherstarrung Legierungs-Partikel einer Länge von ca. 30 mm und einer Dicke von ca. 0,1 mm gebildet. Die sich von der Kühl-Oberfläche lösenden in Rotationsrichtung wegfliegenden Legierungs-Partikel wurden gesammelt.

Untersuchung der Vorlegierungen hinsichtlich ihrer mechanischen Eigenschaften

Die in den Beispielen 1 bis 5 hergestellten Vorlegierungen wurden auf ihre Schüttdichte, ihre mittlere Partikelgröße und ihre Transporteigenschaften hin untersucht. Die Schüttdichte wurde durch Wägung eines definierten Volumens (2 L) an Vorlegierung bestimmt. Für die Abschätzung der Rieselfähigkeit wurde die Vorlegierung unter leichtem Schütteln aus dem Messgefäß geschüttet. Bei deutlicher Verfilzung konnte der Gefäß-Inhalt nur als Ganzes ausgeschüttet werden. Bei bedingter Rieselfähigkeit bzw. bei Rieselfähigkeit konnten einzelne Partikel-Agglomerate bzw. freifließend einzelne Partikel abgeschüttet werden. Die mittlere Partikelgröße wurde durch wiederholte Messung der Partikel mit einem Messschieber (Breite, Länge) bzw. durch elektronenmikroskopische Schnitt-Aufnahmen (Dicke) ermittelt.

Die Ergebnisse der Untersuchungen sind in Tabelle 1 zusammengefasst:

**Tabelle 1**

| **Beispiel** | **Legierung** | **Schüttdichte** | **Mittl. Partikelgröße** | **Transport-Eigenschaft** |
|---|---|---|---|---|
| 1 | Al80Ni17Fe3 rascherstarrt durch | 0,32 | 0,1 x 0,2 x 28 - | rieselfähig |
| | Melt-Overflow-Verfahren (mit Quer- | kg/ L | mm | - keine Verfilzung |
| | Rillen, erfindungsgemäß) | | | |
| 1b | Al71Ni26Fe3 rascherstarrt durch | 0,38 | 0,1 x 0,2 x 26 - | rieselfähig |
| | Melt-Overflow-Verfahren | kg/l | mm | - keine Verfilzung |
| | (mit Quer-Rillen, erfindungsgemäß) | | | |
| 2 | Al80Ni17Fe3 rascherstarrt | 0,30 | 0,1 x 0.1 x 24 | -rieselfähig |
| | durch Schmelzextraktion | kg/ L | mm | - keine Verfilzung |
| | (mit Quer-Rillen, erfindungsgemäß) | | | |
| 3a | Al80Ni17Fe3 rascherstarrt | 0,06 | 0,1 x 25 x 112 - | nicht rieselfähig |
| | durch Guss auf Walze (ohne Quer- | kg/ L | mm | - deutliche Verfilzung |
| | Rillen, nicht erfindungsgemäß) | | | |
| 3b | Al80Ni17Fe3 rascherstarrt | 0,08 | 0,1 x 3 x 84 | - nicht rieselfähig |
| | durch Guss auf Walze (ohne Quer- | kg/ L | mm | - deutliche Verfilzung |
| | Rillen, nicht erfindungsgemäß) | | | |
| 4 | Al80Ni17Fe3 rascherstarrt durch | 0,26 | 0,1 x 3 x 27 | - bedingt rieselfähig |
| | Guss auf Walze (mit Quer-Rillen, er- | kg/ L | mm | - keine Verfilzung |
| | findungsgemäß) | | | |
| 5 | Al71Ni26Fe3 rascherstarrt durch | 0,35 | 0,1 x 3 x 22 | - bedingt rieselfähig |
| | Guss auf Walze (mit Quer-Rillen, er- | kg/ L | mm | - keine Verfilzung |
| | findungsgemäß) | | | |

### Herstellung eines Katalysators aus den Vorlegierungen

### Beispiel 6

782 g Natriumhydroxid wurden in 3129 g Wasser gelöst und die Temperatur der erhaltenen Natronlauge auf 80°C eingestellt. Unter Stickstoffüberlagerung wurden 200 g der zerkleinerten pulverförmigen Ausgangslegierung so zu der Natronlauge gegeben, dass die Temperatur bei 80±2°C gehalten wurde und die Schaumbildung nicht zu stark wurde. Das Reaktionsgemisch wurde 30 Minuten bei 80°C nachgerührt. Im Anschluss wurde die überstehende Lauge abdekantiert und der Rückstand mit einer Lösung von 78 g Natriumhydroxid in 313 g Wasser 5 Minuten unter Rühren nachbehandelt. Auch diese Lauge wurde dekantiert und der Katalysator wurde mit Wasser bis zu einem pH-Wert von 8 bis 9 gewaschen. Der Katalysator wurde quantitativ als wässriger Schlamm erhalten.

### Hydrierung von Dinitrotoluol (DNT)

Verwendet wurde ein Autoklav mit 1000 ml Volumen, ausgestattet mit einem Begasungsrührer, einer Wasserstoffzuführungsleitung, einem Einleitungsrohr für die Nitroverbindung und einem Auslassventil für überschüssigen Wasserstoff. Das Reaktionsgemisch verließ den Reaktor durch eine Fritte, die den Katalysator zurückhielt. Die Temperatur im Reaktor wurde durch einen äußeren Heiz- bzw. Kühlkreislauf geregelt. Eine Kühlschlange im Inneren des Reaktors sorgte für zusätzliche Kühlung des Reaktionsgemisches. 480 g eines technischen Gemisches aus einem Gemisch aus 80% 2,4-Diaminotoluol und 20% 2,6-Diaminotoluol (TDA) mit Wasser im Gewichtsverhältnis von TDA zu Wasser von 63:37 sowie 5 g Katalysator wurden im Reaktor vorgelegt. Anschließend wurde der Reaktorinhalt mit Wasserstoff unter Druck gesetzt und aufgeheizt. Bei einer Temperatur von 180°C und einem Druck von 26 bar wurden stündlich 125 g/h eines technischen Gemisches aus 80% 2,4-Dinitrotoluol und 20% 2,6-Dinitrotoluol in den Reaktor geleitet und bis zur Erschöpfung des Katalysators hydriert. Das resultierende Hydrierungsprodukt wurde kontinuierlich aus dem Reaktor entnommen und zum reinen Diaminotoluol-Isomerengemisch aufgearbeitet.

In einer Versuchsreihe wurden verschiedene Raney-Nickel-Katalysatoren eingesetzt. Das Ergebnis ist in Tabelle 2 dargestellt. Dabei sind die eingesetzten Katalysatoren in Tabelle 2 durch die Zusammensetzung ihrer Vorlegierung und die Art ihrer Rascherstarrung gekennzeichnet. Aus den so erzeugten Vorlegierungen wurden anschließend analog zu Beispiel 6 Raney-Nickel-Katalysatoren hergestellt. Beispiele 7 und 8 wurden dabei mit nicht erfindungsgemäß hergestellten Katalysatoren und Beispiele 9 bis 12 mit erfindungsgemäß hergestellten Katalysatoren durchgeführt.

Der Vergleich der Beispiele 7, 8, 9 und 10 zeigt dabei, dass die erfindungsgemäß hergestellten Katalysatoren gleicher Zusammensetzung in der Hydrierung von DNT höhere Ausbeuten an Toluylendiamin und höhere Standzeiten erreichen.

**Tabelle 2**

| **Beispiel** | **Legierung** | **Struktur / Herstellung des Katalysators** | **TDA-Ausbeute % d. Theorie** | **Standzeith** |
|---|---|---|---|---|
| 7 | Al80Ni17Fe3 (entspricht Beispiel 3a) | Feinkristallin / Rascherstarrung auf unstrukturierter Kühlwalze | 97.6 | 128 |
| 8 | Al80Ni17Fe3 (entspricht Beispiel 3b) | Feinkristallin / Rascherstarrung auf unstrukturierter Kühlwalze | 98.0 | 78 |
| 9 | Al80Ni17Fe3 (entspricht Beispiel 4) | Feinkristallin / Rascherstarrung auf strukturierter Kühlwalze | 98.4 | 153 |
| 10 | Al80Ni17Fe3 (entspricht Beispiel 2) | Feinkristallin / Rascherstarrung durch Schmelzextraktion (durch Quer-Rillen strukturierte Kühlwalze) | 98.2 | 240 |
| 11 | Al71Ni26Fe3 | Feinkristallin / Rascherstarrung auf strukturierter Kühlwalze | 98.4 | 248 |
| 12 | Al71Ni26Fe3 (entspricht Beispiel 1b) | Feinkristallin / Rascherstarrung durch Melt-Overflow-Verfahren (durch Quer-Rillen strukturierte Kühlwalze) | 98.1 | 210 |

## Patentansprüche

1. Verfahren zur Herstellung von Raney-Nickel-Katalysatoren, bei dem man die Schmelze einer Legierung, enthaltend 40 bis 95 Gew.-% Aluminium, 5 bis 50 Gew.-% Nickel, sowie 0 bis 20 Gew.-% Eisen, 0 bis 15 Gew.-% Cer, Cer-Mischmetall, Vanadium, Niob, Tantal, Chrom, Molybdän und/oder Mangan sowie gegebenenfalls weitere glasbildende Elemente mit einer oder mehreren rotierenden Kühlwalzen oder Kühlscheiben in Kontakt bringt, darauf abkühlen und erstarren lässt, wobei die Kühlwalzen eine durch Querrillen und die Kühlscheiben eine durch von der Rotationsachse nach außen verlaufende Rillen strukturierte Oberfläche aufweisen, und man die rasch erstarrte Legierung anschließend einer Behandlung mit organischen oder anorganischen Basen unterzieht.

2. Verfahren zur Herstellung von Raney-Nickel-Katalysatoren nach Anspruch 1, bei dem man die Schmelze nach dem Melt-Overflow-Verfahren aus einem Schmelztiegel auf eine rotierende Kühlwalze abfließen und darauf abkühlen und erstarren lässt, wobei die Oberfläche der Kühlwalze durch Quer-Rillen strukturiert ist.

3. Verfahren zur Herstellung von Raney-Nickel-Katalysatoren nach Anspruch 1, bei dem man aus der Schmelze nach dem Schmelzextraktionsverfahren Teile der Schmelze durch eine in die Schmelze eintauchende rotierende Kühlwalze austrägt und darauf abkühlen und erstarren lässt, wobei die Oberfläche der Kühlwalze durch Quer-Rillen strukturiert ist.

4. Verfahren zur Herstellung von Raney-Nickel-Katalysatoren nach Anspruch 1, bei dem man die Schmelze auf eine rotierende Kühlwalze oder in den Spalt zwischen zwei gegensinnig rotierenden Kühlwalzen gießt und darauf abkühlen und erstarren lässt, wobei die Oberfläche der Kühlwalzen durch Quer-Rillen strukturiert ist.

5. Verfahren zur Herstellung von Raney-Nickel-Katalysatoren nach Anspruch 1, bei dem man die Schmelze auf eine rotierende Kühlscheibe gießt und darauf abkühlen und erstarren lässt, wobei die Oberfläche der Kühlscheibe durch von der Rotationsachse nach außen verlaufende Rillen strukturiert ist.

6. Raney-Nickel-Katalysatoren erhältlich nach dem Verfahren nach einem der Ansprüche 1 bis 5.

7. Verwendung eines Raney-Nickel-Katalysators gemäß Anspruch 6 zur Hydrierung von organischen Verbindungen.

8. Verwendung gemäß Anspruch 7 zur Hydrierung aromatischer Nitroverbindungen.

9. Verwendung gemäß Anspruch 8 zur Hydrierung von 2,4-/2,6-Dinitrotoluolgemischen.

10. Verfahren zur Hydrierung aromatischer Nitroverbindungen, bei dem Raney-Nickel-Katalysatoren gemäß Anspruch 6 eingesetzt werden.
